# FASCICULE DE BREVET EUROPEEN

(11) **EP 4 392 963 B1**
(45) Date de publication et mention de la délivrance du brevet: **06.11.2024**
(21) Numéro de dépôt: 22776969.2
(22) Date de dépôt: 30.08.2022
(51) Int. Cl.: G09B 19/00, A63B 21/068, A63B 22/00, A63B 71/06, G16H 20/30, G16H 40/63, A63B 71/00

(54) **DISPOSITIF POUR LA MISE EN OEUVRE D'UNE TECHNIQUE DE REPRESENTATION MENTALE POUR LA REEDUCATION DE MEMBRES INFERIEURS**
VORRICHTUNG ZUR UMSETZUNG EINER MENTALEN DARSTELLUNGSTECHNIK FÜR DIE REHABILITATION UNTERER GLIEDMASSEN
DEVICE FOR IMPLEMENTING A MENTAL REPRESENTATION TECHNIQUE FOR THE REEDUCATION OF LOWER LIMBS

(30) Priorité: 29.09.2021 FR 2110263
(43) Date de publication de la demande: 03.07.2024
(73) Titulaire: Dessintey, 42650 Saint-Jean-Bonnefonds (FR)
(72) Inventeur: FOURNIER, Nicolas, 42230 SAINT-VICTOR-SUR-LOIRE (FR); LUNEAU, Davy, 43590 BEAUZAC (FR); LAGNIEN, Emmanuel, 07130 SAINT-PERAY (FR)
(74) Mandataire: Cabinet Laurent & Charras
(86) Numéro de dépôt international: PCT/FR2022/051624
(87) Numéro de publication internationale: WO 2023/052695

(56) Documents cités:
- FR-A1- 3 071 723
- FR-A1- 3 071 723
- GB-A- 2 436 150
- KR-B1- 101 196 960
- KR-B1- 101 698 244
- KR-B1- 101 698 244
- ANONYMOUS: "Dessintey unveils its new IVS3 for Lower Limbs", 30 October 2021 (2021-10-30), pages 1 - 1, XP055933076, Retrieved from the Internet <URL:https://pbs.twimg.com/media/FC76PHTX0AMmgDG?format=jpg&name=4096x4096> [retrieved on 20220620]
- ANONYMOUS: "INTENSIVE VISUAL SIMULATION - A UNIQUE TECHNOLOGY DEDICATED TO MOTOR PLANNING AND CENTRAL CONTROL OF MOVEMENT", JUIN, 30 June 2020 (2020-06-30), pages 1 - 23, XP055933063, Retrieved from the Internet <URL:https://www.emac.it/wp-content/uploads/2021/07/DESSINTEY-IVS3-Presentation-2020-EN.pdf> [retrieved on 20220620]

## Description

### Domaine technique

L'invention se rapporte au domaine technique des équipements de réhabilitation et rééducation neuromotrice.

### Art antérieur

Il est connu de l'état de la technique des appareils de mise en oeuvre de techniques de représentation mentale pour la réhabilitation et la rééducation neuromotrice. Ces techniques sont généralement basées sur la sollicitation des neurones visuomoteurs, et peuvent être des simulations ou des entrainements visuomoteurs, tels que des thérapies miroirs par exemple. Ces techniques consistent à faire croire au cerveau qu'un membre déficient est sain par un système d'illusion d'optique.

Classiquement, un miroir peut être utilisé de sorte que lorsqu'un patient regarde une réflexion de son membre sain dans ce miroir, le patient ait l'impression de mouvoir son membre déficient. Ainsi, grâce à la plasticité du cerveau, la re-modélisation de connexions neuronales est possible, avec des résultats avantageux pour remédier à des douleurs chroniques, ou à une déficience motrice du membre. Un exemple de dispositif pour la thérapie miroir est décrit dans le brevet GB2436150.

Plus récemment, des appareils électroniques ont été développés pour les techniques de représentation mentale, en particulier des thérapies miroirs. Ces appareils comprennent une caméra, un écran et un système de support, pour assurer une bonne position relative entre les différents éléments de l'appareil et le patient. Ainsi, la réflexion d'un membre est remplacée par une image modifiée numériquement. De tels dispositifs sont divulgués par exemple dans les documents KR101196960

FR3071723, KR101698244, la présentation "INTENSIVE VISUAL SIMULATION - A UNIQUE TECHNOLOGY DEDICATED TO MOTOR PLANNING AND CENTRAL CONTROL OF MOVEMENT", Juin, 30 juin 2020 (2020-06-30), pages 1-23, XP055933063
ou encore dans le brevet EP3692543 du Demandeur.

Toutefois ces appareils sont plutôt adaptés pour les membres supérieurs uniquement. Certains appareils peuvent capter des images des mouvements des membres inférieurs sains, notamment d'un point de vue plongeant, mais les résultats des thérapies effectuées avec ces appareils ne donnent pas entière satisfaction.

De plus, l'appareil doit être réglé en fonction de la morphologie de chaque patient, notamment selon sa carrure, son maintien, sa posture et ses possibilités de mouvement, influencés par ses déficiences, son handicap, on encore par l'utilisation d'un fauteuil roulant. Du fait du temps relativement long pour l'installation du patient à chaque séance, l'efficacité du traitement s'en trouve diminué.

### Exposé de l'invention

L'un des buts de l'invention est de pallier les inconvénients de l'art antérieur en proposant un équipement de réhabilitation et rééducation neuromotrice basé sur les techniques de représentation mentale, qui soit adapté pour les membres inférieurs, et qui fournisse des résultats améliorés par rapport à ceux de l'art antérieur.

Un autre but de l'invention est de proposer un appareil qui sera simple à mettre en oeuvre, avec une facilité de réglage.

À cet effet, il a été mis au point un dispositif de mise en oeuvre d'une technique de représentation mentale pour la rééducation d'au moins un membre inférieur, face auquel un patient est destiné à s'installer, le dispositif comprenant :
- une caméra centrale disposée de sorte à filmer le membre inférieur selon un point de vue central ;
- un écran central d'affichage disposé de sorte à masquer la vue directe du patient sur le membre inférieur, et destiné à afficher le point de vue filmé par la caméra centrale.

Selon l'invention, le dispositif comprend également au moins une caméra latérale disposée de sorte à filmer latéralement le membre inférieur, et un écran d'affichage latéral destiné à afficher le point de vue filmé par la caméra latérale.

De cette manière, le point de vue central, nécessaire pour que le patient s'approprie les images projetées sur l'écran central, est complété par un deuxième point de vue latéral qui permet de mieux visualiser les mouvements effectués. Toute l'amplitude des mouvements effectués par le membre inférieur est perceptible, ce qui permet de solliciter au mieux les neurones visuomoteurs, et ainsi de rendre les exercices plus efficaces. En effet, les mouvements des muscles releveurs, qui permettent de fléchir le pied vers le haut et vers le bas, et dont la mobilisation est essentielle pour le réapprentissage de la marche, sont bien perçus par le patient.

Dans un mode de réalisation préféré, le dispositif comprend deux caméras latérales, disposées de part et d'autre de la caméra centrale, et deux écrans latéraux disposés de part et d'autre de l'écran central. De cette manière le dispositif est symétrique et permet de filmer et de restituer les images du membre inférieur droit ou gauche du patient sans qu'il y ait besoin de déplacer de matériel, ce qui ferait perdre du temps.

Afin de s'adapter facilement à la morphologie du patient, la caméra centrale ainsi que l'écran central sont reliés à une structure, et la structure est réglable en hauteur. Ce réglage en hauteur permet également de s'adapter à une station assis ou debout du patient.

En vue d'améliorer l'ergonomie du système et le confort du patient, la structure comprend une tablette, destinée à recevoir en appui des membres supérieurs du patient.

Le dispositif comprend une interface de pilotage et de contrôle, accessible à un praticien. Ainsi, le dispositif est complet et autonome. S'il comporte une structure montée sur roulettes, le dispositif complet peut être déplaçable, et remis en route rapidement sans perdre de temps à reconnecter une nouvelle interface de contrôle.

De préférence, les roulettes sont verrouillables par des freins ou escamotables dans une position hors-contact du sol. De cette manière, le positionnement du dispositif est verrouillable.

Pour faire coïncider au mieux le point de vue du patient et l'affichage de l'écran central, que le patient soit en station assis ou debout, celui-ci est mobile en translation par rapport à la structure selon un axe antéro-postérieur (S) du patient, de préférence également selon un axe transversal du patient, de préférence mobile le long d'un arc de cercle centré sur le patient, et encore préférentiellement en pivotement autour d'un axe parallèle à l'axe transversal du patient. Pour le patient, cela renforce l'illusion que l'affichage de l'écran central correspond à ses membres qu'il regarde directement.

Pour limiter les déformations d'image et renforcer l'immersion visuelle, l'écran central est incurvé autour d'un axe parallèle à un axe transversal du patient.

Le dispositif peut comprendre en outre une estrade destinée à recevoir au moins un pied du patient, et de préférence l'estrade est inclinable et/ou réglable en hauteur. Cette estrade permet entre autres au dispositif d'être polyvalent et de pouvoir accueillir des patients pouvant se déplacer avec fauteuil roulant ainsi que sans fauteuil roulant.

Pour diversifier les exercices de rééducation possibles, l'estrade comprend des dispositifs d'exercice de rééducation destinés à coopérer avec le pied posé sur l'estrade, présentant de préférence des effets visuels, tels que des zones colorées et/ou lumineuses, et/ou des effets haptiques, tels qu'une surface rugueuse, une tôle larmée, des stries, et/ou des objets déplaçables éventuellement équipés d'un aimant destiné à se fixer sur une zone métallique de l'estrade.

Toujours dans un but de polyvalence entre les patients et pour pouvoir effectuer différents exercices, l'estrade est de préférence escamotable et peut être démontée du dispositif.

Afin que d'assister le patient lors de son maintien en station debout, le dispositif comprend une barre de maintien reliée à l'ensemble mobile, de préférence la barre de maintien est escamotable entre une position repliée, et une position déployée où la barre fait saillie du dispositif. Le dispositif peut donc aussi bien être utilisé en station assis que debout.

### Brève description des dessins

[Fig.1] est une vue en perspective, vue de dessus, d'un premier dispositif selon l'invention.
[Fig.2] est une vue de côté d'un deuxième dispositif selon l'invention.
[Fig.3] est une vue de face du dispositif.
[Fig.4] est une vue d'un troisième dispositif selon l'invention, en cours d'utilisation.
[Fig.5] est une autre vue du dispositif en cours d'utilisation.
[Fig.6] est une vue d'une estrade du dispositif.
[Fig.7] est un schéma anatomique.
[Fig.8] est un schéma illustrant un pied vu de dessus.
[Fig.9] est un schéma illustrant un pied en dorsiflexion vu de dessus.
[Fig.10] est un schéma illustrant les mouvements d'un pied en dorsiflexion et en plantiflexion vus de côté.

### Description détaillée de l'invention

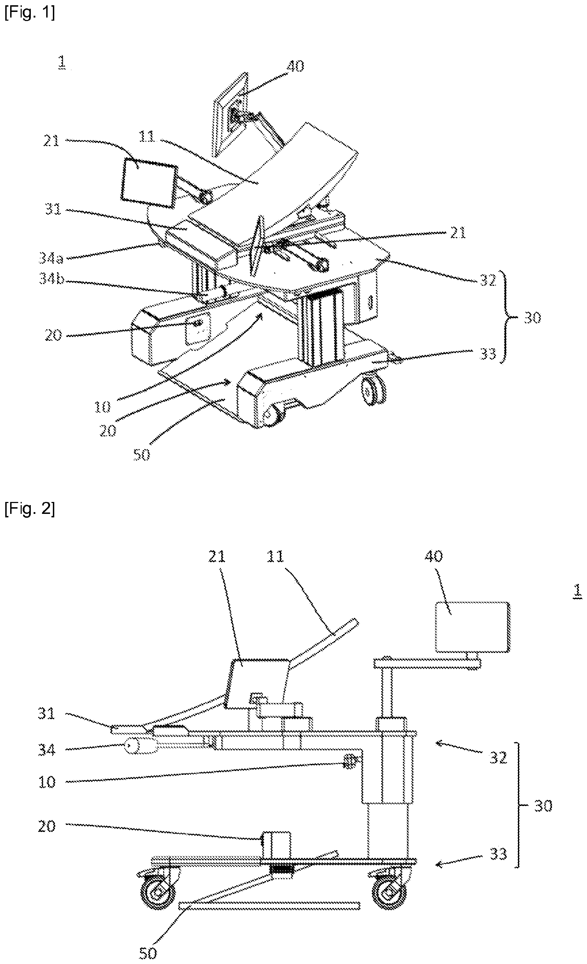

En référence aux figures 1 à 3, l'invention est un dispositif destiné à la stimulation visuomotrice, utilisé lors de thérapies des membres inférieurs, telles que des thérapies miroirs. Dans la description ci-après, le "membre inférieur" désigne la cuisse et/ou la jambe et/ou le pied du patient.

A cet effet, le dispositif comprend une caméra centrale (10) projetant une image sur un écran central (11), ainsi qu'au moins une caméra latérale (20) projetant une image sur un écran latéral (21). Les caméras centrale et latérales (10, 20) et les écrans central et latéraux (11, 21) sont supportés par une structure (30) du dispositif.

Cette structure (30) comprend avantageusement une tablette (31) sur laquelle le patient peut reposer ses bras durant la séance, afin d'améliorer son confort. En effet, lorsqu'un patient a besoin d'un traitement pour un membre inférieur, il y a une probabilité importante qu'il souffre également d'une atteinte à un membre supérieur. Ceci est particulièrement le cas lorsque le patient a subi un accident vasculaire cérébral, ou souffre d'une maladie dégénérative du type maladie de Parkinson ou sclérose en plaques. Il y a donc besoin de prendre en charge le membre supérieur, et la tablette (31) constitue un support adapté pour les bras. De préférence, la position de la tablette (31) est réglable afin de s'adapter à la morphologie du patient. Il peut s'agir d'une translation selon un axe parallèle à un axe antéro-postérieur (S) du patient, ou à un axe transversal (T) du patient, ou encore à une combinaison de plusieurs translations.

Par ailleurs, cette tablette (31) permet de limiter l'envie du patient de regarder par les côtés de l'écran central (11) pour vérifier ce qu'il se passe vraiment au niveau de ses membres inférieurs, ce qui casserait l'effet de la thérapie.

De préférence, la structure (30) comprend un système de réglage en hauteur de manière à pouvoir ajuster simultanément la hauteur de la caméra centrale (10), de l'écran central (11) et de la tablette (31). Ces éléments sont donc montés sur un ensemble mobile (32), qui peut se déplacer verticalement par rapport à une base (33) à l'aide de tout moyen adapté tel qu'un vérin électrique. Le dispositif (1) est donc facile à adapter à différentes morphologies de patients.

De plus, le dispositif (1) peut comprendre une estrade (50), afin que ces patients puissent reposer leurs pieds sur ladite estrade (50). L'estrade (50) est de préférence escamotable ou amovible de manière que le dispositif (1) soit polyvalent. Cette estrade (50) peut servir par exemple si un patient est en fauteuil roulant.

En partie inférieure, le dispositif (1) est le plus dégagé possible afin de laisser au praticien un accès libre aux membres inférieurs, à l'estrade (50) si elle est présente, et à d'éventuels dispositifs supplémentaires utilisés pendant les exercices.

Afin que la stimulation visuomotrice soit la plus efficace possible, l'écran central (11) doit donner au patient l'impression qu'il regarde directement ses membres inférieurs, comme s'il n'y avait ni caméra centrale (10) ni écran central (11).

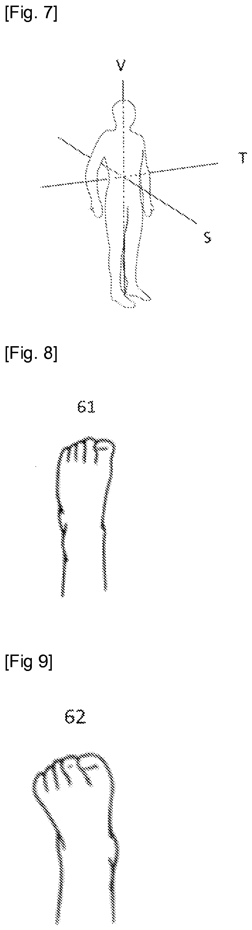

Pour ce faire, l'écran central (11) est de préférence réglable en position par rapport à l'ensemble mobile (32). Avantageusement, l'écran central (11) est mobile en translation par rapport à l'ensemble mobile (32) selon l'axe antéro-postérieur (S) du patient ainsi que selon l'axe transversal (T) du patient, de préférence mobile le long d'un arc de cercle centré sur le patient, et encore préférentiellement en pivotement autour d'un axe parallèle à l'axe transversal (T) du patient. Par souci de clarté, l'axe antéro-postérieur (S), l'axe transversal (T) et l'axe longitudinal (V) du patient sont rappelés sur le schéma de la figure 7.

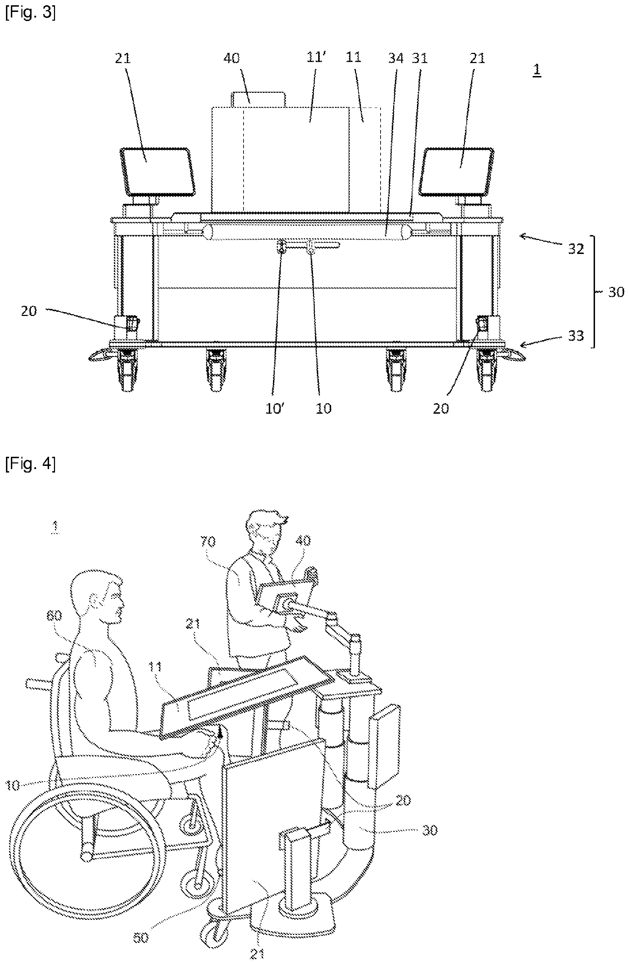

La translation selon un axe transversal (T) de la caméra centrale (10) et de l'écran central (11) est visible sur la figure 3, où ces deux éléments ont été amenés à l'extrême gauche, respectivement en positions (10') et (11').

La possibilité de régler la position transversale de la caméra centrale (10), et si possible de l'écran central (11) de manière concomitante, est avantageuse car cela permet de faire une captation du membre sain uniquement. En effet, le travail cérébral doit être latéralisé, afin que la partie saine du cerveau ne prenne pas le dessus sur la partie lésée. En ne filmant puis en ne faisant travailler qu'un seul membre à la fois, avec un seul membre affiché à la fois, on facilite cette latéralisation permettant de mieux cibler les zones du cerveau à rééduquer.

Au surplus, dans le cas particulier d'un patient amputé, souffrant par exemple de douleurs du membre fantôme, cela permet de ne faire une captation que du membre restant, et de ne pas filmer le moignon. Sinon, l'écran central (11) afficherait par la suite une image inversée où le patient verrait son membre valide être virtuellement amputé, ce qui pourrait être traumatisant.

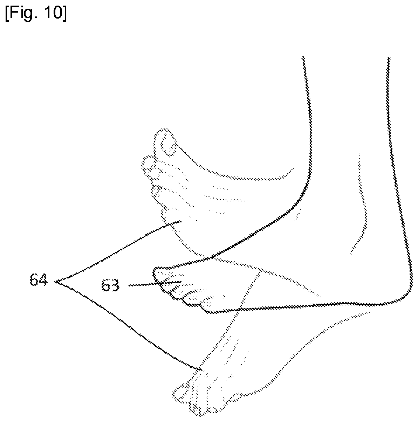

Les écrans latéraux (21), même s'ils ne correspondent pas à un point de vue direct du patient, permettent de mieux percevoir le mouvement effectué. En référence à la figure 10, tout l'amplitude des mouvements de plantiflexion et de dorsiflexion est bien visible, ce qui accentue la stimulation des neurones visuomoteurs par rapport à un point de vue plongeant tel qu'illustré aux figures 8 et 9.

De préférence, le dispositif (1) comprend deux caméras latérales (20) et deux écrans latéraux (21), disposés de chaque côté du dispositif (1). De cette manière, il n'y a pas besoin de déplacer du matériel lorsqu'il faut passer du traitement d'un membre gauche à un membre droit ou inversement.

De plus, un écran latéral (21) peut être utile lorsque le patient enregistre les mouvements de son membre sain en début de séance : il peut vérifier s'il effectue les gestes correctement.

Avoir un deuxième écran latéral (21) permet aussi de gagner du temps pour démarrer la phase de travail lorsque les mouvements enregistrés du côté du membre valide sont ensuite affichés du côté du membre pathologique, puisqu'il n'y a aucune manipulation mécanique à faire.

En référence à la figure 4, on voit un dispositif (1) en cours d'utilisation par un patient (60), la séance étant guidée par un praticien (70). Le praticien (70) dispose d'une interface (40) de pilotage et de contrôle, au travers de laquelle il peut piloter les moteurs du dispositif (1), par exemple ceux du réglage en hauteur de l'ensemble mobile (32) par rapport à la base (33). Il utilise également cette interface (40) pour piloter l'enregistrement des différents mouvements effectués avec le membre sain, avant d'afficher l'image inversée pour que le patient effectue ses exercices de stimulation visuomotrice. A cet effet, l'interface (40) peut être reliée à un ordinateur présent dans le dispositif (1). L'interface (40) peut comprendre par exemple un écran tactile, ou encore des boutons disposés sur la structure (30). Il est entendu que le moniteur de l'interface (40) n'est pas un écran (11, 21) au sens de l'invention : en effet, le moniteur de l'interface n'a pas vocation à permettre au patient de visualiser les mouvements des membres.

Une séance classique de stimulation visuomotrice se déroule selon les étapes décrites ci-après.

La première étape est l'installation du patient devant le dispositif (1). Le dispositif (1) est ajusté en fonction de la morphologie du patient, afin que celui-ci soit à l'aise : il doit pouvoir reposer ses bras sur la tablette (31), ses pieds sur l'estrade (50) le cas échéant, la caméra centrale (10) est réglée afin de cadrer le membre sain, et l'écran principal (11) est positionné de sorte que l'affichage des membres inférieurs soit aligné avec les membres du patient.

La deuxième étape est la captation des mouvements effectués par le membre sain, et leur enregistrement au sein d'une base de données informatique du dispositif (1). Les mouvements sont de différentes natures et sont exécutés à la demande du praticien en fonction des muscles à remobiliser ou de la thérapie prévue. Il est important de faire des mouvements lents et de bien décomposer les gestes afin de faire un modèle plus facile à interpréter et à suivre lors de la troisième étape.

La troisième étape est la stimulation visuomotrice en tant que telle. L'affichage de l'écran principal (11) et des écrans latéraux (21) est modifié de sorte à leurrer le cerveau du patient et lui faire croire que c'est le membre pathologique qui exécute les mouvements enregistrés. En pratique, dans le cadre d'une thérapie miroir :
- l'affichage de l'écran central (11) est réfléchi selon une direction horizontale (inversion de la droite et de la gauche) ; et
- l'affichage du premier écran latéral (21) est échangé avec l'affichage du second écran latéral (21), avec les éventuelles réflexions nécessaires selon les dispositions desdits écrans.

La stimulation visuomotrice est donc un processus en plusieurs étapes dissociées. L'étape de captation est critique étant donné l'importance d'enregistrer des mouvements suffisamment lents et lisibles par le cerveau : si les mouvements sont trop rapides ou si leur amplitude est faible, la stimulation visuomotrice ne sera pas efficace. Les écrans latéraux (21) permettant de mieux voir les membres inférieurs en cours de travail, par exemple les mouvements de dorsiflexion et plantiflexion du pied, le dispositif (1) selon l'invention permet d'améliorer l'efficacité de la stimulation.

On voit également sur la figure 4 que le dispositif (1) est autonome : la structure (30) porte tous les éléments électroniques, de sorte que le dispositif (1) est compact. Avantageusement, la base (33) est montée sur des roulettes de sorte que le dispositif (1) est mobile. Cette mobilité peut être mise à profit pour déplacer le dispositif (1) au sein d'une salle de soins, par exemple pour adapter la position du dispositif (1) aux conditions de luminosité ou encore pour organiser les soins à pratiquer sur un plateau de soins comprenant différents types de dispositifs. Cette mobilité peut également servir afin d'ajuster la position du dispositif (1) par rapport au patient lors de l'étape d'installation. Afin de pouvoir ensuite verrouiller la position du dispositif (1), les roulettes sont pourvues de freins, ou encore les roulettes peuvent être escamotables entre une position en contact avec le sol et permettant la mobilité du dispositif (1), et une position hors contact du sol où le dispositif (1) repose directement sur le sol. En alternative, le dispositif (1) comprend des pieds mobiles entre une position haute où ils ne touchent pas le sol, laissant le dispositif (1) reposer sur les roulettes dans une position de mobilité, et une position basse où les pieds sont en contact avec le sol et escamotent les roulettes en position hors-contact. Par ailleurs, les roulettes permettent aussi d'enlever le dispositif (1) en fin de séance, afin que le patient puisse investir ses membres en exécutant les mêmes exercices en vision directe.

Au surplus, la présence de roulettes permet également d'envisager des exercices où le patient marche en suivant le dispositif (1). Dans ce cas, le dispositif (1) comprend avantageusement des moyens de stockage d'énergie, de sorte qu'il puisse être utilisé en autonomie et sans être relié à une prise de courant.

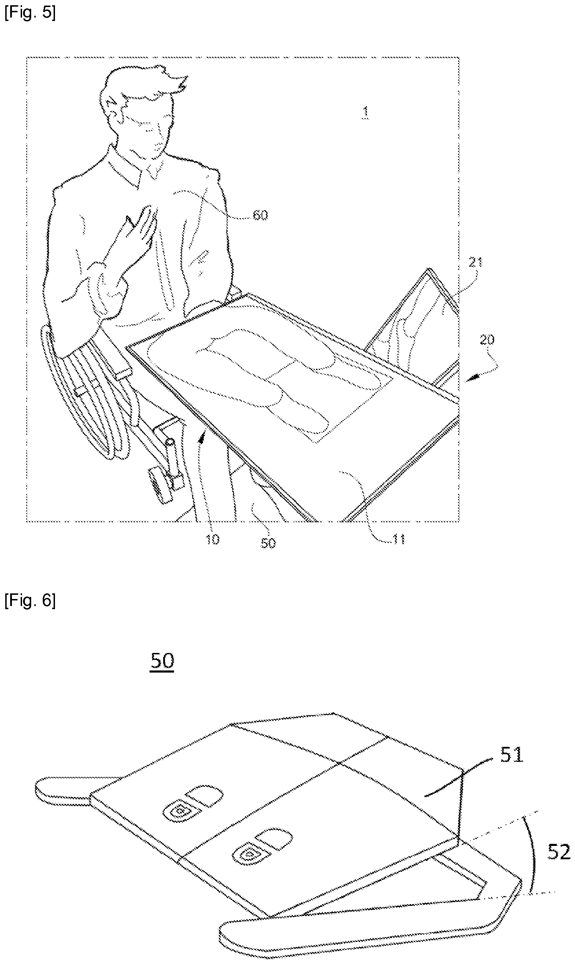

Dans le mode de réalisation illustré à la figure 5, la caméra centrale (10) et l'écran central (11) sont réglés de sorte à reproduire le point de vue du patient, qui croit regarder ses propres membres inférieurs et non pas un affichage.

Dans le mode de réalisation illustré aux figures 1 à 3, la caméra centrale (10) est repoussée vers l'arrière du dispositif (1). Cette disposition ne permet pas de créer un affichage se substituant au point de vue du patient, néanmoins la captation effectuée est focalisée sur les parties en mouvement des membres plutôt que sur les cuisses, qui sont généralement immobiles. Bien que le point de vue du patient ne soit pas exactement reproduit, la perception des mouvements est meilleure, ce qui favorise l'efficacité de la thérapie. L'alignement de l'affichage de l'écran central (11) et des membres du patient lui permet tout même d'identifier le membre affiché comme étant le sien.

La figure 6 illustre une estrade (50) multifonction. Cette estrade (50) permet d'ajuster le réglage en hauteur d'une surface de réception (51) des pieds.

L'estrade (50) permet aussi de faciliter le déclenchement du mouvement pour des muscles releveurs, lorsqu'elle présente une surface de réception (51) inclinée. Cette surface inclinée permet aussi de rattraper un différentiel de hauteur de réception du pied, en modifiant la profondeur de l'estrade par rapport au patient : on peut ajuster finement la hauteur de réception du pied sans utiliser de réglage mécanique ou de motorisation. Avantageusement, l'angle d'inclinaison (52) de la surface de réception (51) est compris entre 0 et 20°.

La surface de réception (51) peut également présenter différents dispositifs permettant de diversifier, de spécifier, ou de rendre ludiques les exercices de motricité à effectuer :
- Il peut s'agir de présenter des effets visuels, tels que des zones colorées et/ou lumineuses, au moyen d'une surface de réception (51) pourvue par exemple de LED ou d'une dalle d'affichage ;
- Il peut s'agir d'effets haptiques au moyen d'une surface de réception (51) comprenant par exemple des zones rugueuses et d'autres lisses, ou encore présentant des stries ;
- Le patient peut aussi déplacer avec ses pieds par exemple des objets posés sur la surface de réception (51), auquel cas celle-ci peut être en tôle ou en acier de façon à coopérer avec des aimants disposés à la base desdits objets.

Lors de la rééducation, le patient en phase de réapprentissage de la marche peut être amené à effectuer des exercices en station debout plutôt qu'assise. A cet effet, le dispositif (1) comprend une ou plusieurs barres de maintien (34) destinées à être saisies manuellement par le patient. Ces barres (34) peuvent être escamotables tel que représenté à la figure 1 : une première barre (34) est en position rangée (34a), et une seconde barre (34) est en position déployée (34b). Au lieu d'être disposée selon un axe antéro-postérieur tel qu'à la figure 1, une barre (34) peut être transversale, telle que représentée en position rangée aux figures 2 et 3. Ces barres (34) sont donc amenées à être déployées vers l'avant, en direction du patient, afin qu'il puisse les saisir lorsqu'il est en station debout.

Pour ces exercices en station debout, la position de la caméra centrale (10) vers l'arrière du dispositif (1) ainsi que la position des caméras latérales (20), toutes avec un point de vue peu plongeant, permettent de cadrer la captation des pieds au bassin du patient. De plus, l'écran central (11) est réglable selon l'axe antéro-postérieur (S) de sorte que sa position peut être adaptée pour correspondre au mieux au point de vue du patient. En l'espèce, l'écran central (11) est reculé au maximum, dans une position éloignée du patient, permettant ainsi de dégager les barres de maintien (34) que le patient doit saisir. La ou les caméras latérales (20) sont fixes, de manière à toujours être focalisées sur les pieds du patient, qu'il soit en station assis ou debout.

Par ailleurs, le dispositif (1) peut être conformé différemment des figures sans sortir du cadre de l'invention, qui est défini par les revendications.

En variante non représentée, le dispositif (1) ne comporte qu'une seule caméra latérale (20) et qu'un seul écran latéral (21), dans un but d'économie de matériel.

Dans une autre variante non représentée, les barres (34) sont verticales, et sont disposées de part et d'autre du dispositif (1).

Dans une autre variante non représentée, le dispositif (1) comprend des moyens d'éclairage permettant de garantir une bonne luminosité pour la captation des images. Ces moyens d'éclairage sont de préférence disposés sous l'ensemble mobile (32).

Il est également possible, au moyen de l'interface de commande (40), de sélectionner les écrans qui affichent ou pas. Par exemple l'écran central (11) peut être tout le temps activé, alors qu'un seul des écrans latéraux (21) est allumé, etc. En particulier, dans le cas des patients héminégligents, un seul des écrans latéraux (21) est allumé, et de préférence sa position latérale est éloignée du patient. De la sorte, il est possible de forcer l'attention du patient du côté de son héminégligence.

Dans certains cas, les caméras ne font pas de captation et les écrans (central et latéraux) projettent des images enregistrées au préalable. Par exemple dans le cas de patients souffrant d'affections aux deux membres inférieurs, et qui ne disposent pas d'un membre sain avec lesquels enregistrer des mouvements lors de la phase de captation, la stimulation visuomotrice est effectuée à l'aide de mouvements enregistrés par un tiers. Dans ce mode, les caméras ne sont pas utilisées lors de la séance de stimulation visuomotrice, mais les affichage central et latéraux produisent l'effet recherché pour leurrer le cerveau.

En outre, les caractéristiques techniques des différents modes de réalisation et variantes mentionnés ci-dessus peuvent être, en totalité ou pour certaines d'entre elles, combinées entre elles. Ainsi, le dispositif (1) peut être adapté en termes de coût, de fonctionnalités et de performance.

## Revendications

1. Dispositif (1) de mise en oeuvre d'une technique de représentation mentale pour la rééducation d'au moins un membre inférieur, face auquel un patient est destiné à s'installer, le dispositif comprenant :
- une caméra centrale (10) disposée de sorte à filmer le membre inférieur selon un point de vue central ;
- un écran central (11) d'affichage disposé de sorte à masquer la vue directe du patient sur le membre inférieur, et destiné à afficher le point de vue filmé par la caméra centrale (10) ;
***caractérisé en* ce qu**'il comprend également au moins une caméra latérale (20) disposée de sorte à filmer latéralement le membre inférieur, et un écran d'affichage latéral (21) destiné à afficher le point de vue filmé par la caméra latérale (20).

2. Dispositif (1) selon la revendication 1 ***caractérisé en* ce qu**'il comprend deux caméras latérales (20), disposées de part et d'autre de la caméra centrale (10), et deux écrans latéraux (21) disposés de part et d'autre de l'écran central (11).

3. Dispositif (1) selon la revendication 1 ***caractérisé en* ce *que*** la caméra centrale (10) ainsi que l'écran central (11) sont reliés à une structure (30), et la structure (30) est réglable en hauteur.

4. Dispositif (1) selon la revendication 3 ***caractérisé en* ce *que*** la structure (30) comprend une tablette (31) destinée à recevoir en appui des membres supérieurs du patient.

5. Dispositif (1) selon la revendication 1 ***caractérisé en* ce qu**'il comprend une barre de maintien (34) reliée à l'ensemble mobile (32), de préférence la barre de maintien (34) est escamotable entre une position repliée, et une position déployée où la barre (34) fait saillie du dispositif (1).

6. Dispositif (1) selon la revendication 1 ***caractérisé en* ce qu**'il comprend des roulettes, verrouillables par des freins ou escamotables dans une position hors-contact du sol.

7. Dispositif (1) selon la revendication 1 ***caractérisé en* ce qu**'il comprend une interface (40) de pilotage et de contrôle, accessible à un praticien.

8. Dispositif (1) selon la revendication 3, ***caractérisé en* ce *que*** l'écran central (11) est mobile en translation par rapport à la structure (30) selon un axe antéro-postérieur (S) du patient ainsi que selon un axe transversal (T) du patient, de préférence mobile le long d'un arc de cercle centré sur le patient, et encore préférentiellement en pivotement autour d'un axe parallèle à l'axe transversal (T) du patient.

9. Dispositif (1) selon la revendication 1, ***caractérisé en* ce *que*** l'écran central (11) est incurvé autour d'un axe parallèle à un axe transversal (T) du patient.

10. Dispositif (1) selon la revendication 1 ***caractérisé en* ce qu**'il comprend en outre une estrade (50) destinée à recevoir au moins un pied du patient, et de préférence l'estrade (50) est inclinable et/ou réglable en hauteur.

11. Dispositif (1) selon la revendication 9 ***caractérisé en* ce *que*** l'estrade (50) comprend des dispositifs d'exercice de rééducation destinés à coopérer avec le pied posé sur l'estrade (50), présentant de préférence des effets visuels, tels que des zones colorées et/ou lumineuses, et/ou des effets haptiques, tels qu'une surface rugueuse, une tôle larmée, des stries, et/ou des objets déplaçables éventuellement équipés d'un aimant destiné à se fixer sur une zone métallique de l'estrade (50).

12. Dispositif (1) selon la revendication 9 ***caractérisé en* ce *que*** l'estrade (50) est escamotable et peut être démontée du dispositif (1).

## Patentansprüche

1. Gerät (1) zur Durchführung einer mentalen Repräsentationstechnik zur Rehabilitation von mindestens einer unteren Gliedmaße, vor dem ein Patient positioniert werden soll, wobei das Gerät umfasst:
- eine zentrale Kamera (10), die angeordnet ist, um die untere Gliedmaße aus einer zentralen Perspektive zu filmen;
- einen zentralen Bildschirm (11), der angeordnet ist, um die direkte Sicht des Patienten auf die untere Gliedmaße zu blockieren und die von der zentralen Kamera (10) gefilmte Perspektive anzuzeigen; **dadurch gekennzeichnet, dass** es auch mindestens eine seitliche Kamera (20) umfasst, die angeordnet ist, um die untere Gliedmaße aus einer seitlichen Perspektive zu filmen, und einen seitlichen Bildschirm (21), der die von der seitlichen Kamera (20) gefilmte Perspektive anzeigen soll.

2. Gerät (1) nach Anspruch 1, **dadurch gekennzeichnet, dass** es zwei seitliche Kameras (20) umfasst, die auf beiden Seiten der zentralen Kamera (10) positioniert sind, und zwei seitliche Bildschirme (21), die auf beiden Seiten des zentralen Bildschirms (11) positioniert sind.

3. Gerät (1) nach Anspruch 1, **dadurch gekennzeichnet, dass** die zentrale Kamera (10) sowie der zentrale Bildschirm (11) mit einer Struktur (30) verbunden sind, und die Struktur (30) in der Höhe verstellbar ist.

4. Gerät (1) nach Anspruch 3, **dadurch gekennzeichnet, dass** die Struktur (30) ein Regal (31) umfasst, das dazu bestimmt ist, die oberen Gliedmaßen des Patienten zu stützen.

5. Gerät (1) nach Anspruch 1, **dadurch gekennzeichnet, dass** es eine Stützstange (34) umfasst, die mit der mobilen Einheit (32) verbunden ist, vorzugsweise ist die Stützstange (34) zwischen einer eingeklappten Position und einer ausgeklappten Position, in der die Stange (34) aus dem Gerät (1) herausragt, einziehbar.

6. Gerät (1) nach Anspruch 1, **dadurch gekennzeichnet, dass** es Räder umfasst, die durch Bremsen verriegelbar oder in einer vom Boden abgehobenen Position einziehbar sind.

7. Gerät (1) nach Anspruch 1, **dadurch gekennzeichnet, dass** es eine Steuer- und Bedienoberfläche (40) umfasst, die für einen Praktiker zugänglich ist.

8. Gerät (1) nach Anspruch 3, **dadurch gekennzeichnet, dass** der zentrale Bildschirm (11) relativ zur Struktur (30) entlang einer anteroposterioren Achse (S) des Patienten sowie entlang einer transversalen Achse (T) des Patienten beweglich ist, vorzugsweise entlang eines Bogens, der auf den Patienten zentriert ist, und noch vorzugsweise um eine Achse drehbar ist, die parallel zur transversalen Achse (T) des Patienten verläuft.

9. Gerät (1) nach Anspruch 1, **dadurch gekennzeichnet, dass** der zentrale Bildschirm (11) um eine Achse gekrümmt ist, die parallel zur transversalen Achse (T) des Patienten verläuft.

10. Gerät (1) nach Anspruch 1, **dadurch gekennzeichnet, dass** es ferner eine Plattform (50) umfasst, die dazu bestimmt ist, mindestens einen Fuß des Patienten aufzunehmen, und vorzugsweise ist die Plattform (50) neigbar und/oder in der Höhe verstellbar.

11. Gerät (1) nach Anspruch 9, **dadurch gekennzeichnet, dass** die Plattform (50) Rehabilitationsübungsgeräte umfasst, die dazu bestimmt sind, mit dem auf der Plattform (50) platzierten Fuß zu kooperieren, vorzugsweise mit visuellen Effekten, wie farbigen und/oder leuchtenden Bereichen, und/oder haptischen Effekten, wie einer rauen Oberfläche, Riffelblech, Rillen und/oder beweglichen Objekten, die möglicherweise mit einem Magneten ausgestattet sind, der dazu bestimmt ist, sich an einem metallischen Bereich der Plattform (50) zu befestigen.

12. Gerät (1) nach Anspruch 9, **dadurch gekennzeichnet, dass** die Plattform (50) einziehbar ist und vom Gerät (1) demontiert werden kann.

## Claims

1. Device (1) for implementing a mental representation technique for the rehabilitation of at least one lower limb, in front of which a patient is intended to be positioned, the device comprising:
- a central camera (10) arranged to film the lower limb from a central point of view;
- a central display screen (11) arranged to block the patient's direct view of the lower limb, intended to display the viewpoint filmed by the central camera (10); **characterized in that** it also includes at least one lateral camera (20) arranged to film the lower limb from a lateral perspective, and a lateral display screen (21) intended to display the viewpoint filmed by the lateral camera (20).

2. Device (1) according to claim 1 **characterized in that** it includes two lateral cameras (20), positioned on either side of the central camera (10), and two lateral screens (21) positioned on either side of the central screen (11).

3. Device (1) according to claim 1 **characterized in that** the central camera (10) as well as the central screen (11) are connected to a structure (30), and the structure (30) is adjustable in height.

4. Device (1) according to claim 3 **characterized in that** the structure (30) includes a shelf (31) intended to support the patient's upper limbs.

5. Device (1) according to claim 1 **characterized in that** it includes a support bar (34) connected to the mobile assembly (32), preferably the support bar (34) is retractable between a folded position, and a deployed position where the bar (34) protrudes from the device (1).

6. Device (1) according to claim 1 **characterized in that** it includes wheels, lockable by brakes or retractable in an off-ground position.

7. Device (1) according to claim 1 **characterized in that** it includes a control and operating interface (40), accessible to a practitioner.

8. Device (1) according to claim 3, **characterized in that** the central screen (11) is movable in translation relative to the structure (30) along an antero-posterior axis (S) of the patient as well as along a transversal axis (T) of the patient, preferably movable along an arc centered on the patient, and even more preferably pivotable around an axis parallel to the transversal axis (T) of the patient.

9. Device (1) according to claim 1, **characterized in that** the central screen (11) is curved around an axis parallel to a transversal axis (T) of the patient.

10. Device (1) according to claim 1 **characterized in that** it further comprises a platform (50) intended to receive at least one foot of the patient, and preferably the platform (50) is tiltable and/or adjustable in height.

11. Device (1) according to claim 9 **characterized in that** the platform (50) includes rehabilitation exercise devices designed to cooperate with the foot placed on the platform (50), preferably featuring visual effects, such as colored and/or luminous areas, and/or haptic effects, such as a rough surface, checker plate, grooves, and/or movable objects possibly equipped with a magnet intended to attach to a metallic area of the platform (50).

12. Device (1) according to claim 9 **characterized in that** the platform (50) is retractable and can be dismantled from the device (1).
